(19) Europäisches Patentamt
European Patent Office
Office européen des brevets

(11) Veröffentlichungsnummer: **0 499 836 A2**

(12) **EUROPÄISCHE PATENTANMELDUNG**

(21) Anmeldenummer: **92101314.0**

(22) Anmeldetag: **28.01.92**

(51) Int. Cl.5: **C08G 65/32**, C08J 3/03, C07C 69/96

(30) Priorität: **21.02.91 DE 4105355**

(43) Veröffentlichungstag der Anmeldung: **26.08.92 Patentblatt 92/35**

(84) Benannte Vertragsstaaten: **BE CH DE ES FR GB IT LI NL SE**

(71) Anmelder: **BASF Aktiengesellschaft**
**Carl-Bosch-Strasse 38**
**W-6700 Ludwigshafen(DE)**

(72) Erfinder: **Boettcher, Andreas, Dr.**
**Konrad-Adenauer-Ring 38**
**W-6907 Nussloch(DE)**
Erfinder: **Schwartz, Manfred, Dr.**
**Friesenstrasse 24**
**W-6700 Ludwigshafen(DE)**

(54) **Carbonat- und Carbonylgruppen enthaltende Verbindungen, Verfahren zu deren Herstellung und ihre Verwendung.**

(57) Die Erfindung betrifft Carbonat- und Carbonylgruppen enthaltende Verbindungen der allgemeinen Formel

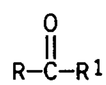

worin

R für einen Alkylrest, für einen Arylrest oder den Rest $R^1$ steht und

$R^1$ für den Rest

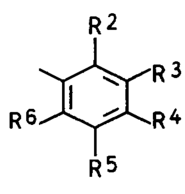

steht, wobei die Reste $R^2$ bis $R^6$ für H, Alkyl, OH, OAlkyl, SH, SAlkyl, Halogen, $N(Alkyl)_2$, N(Alkyl)(Aryl) stehen und mindestens einer aber maximal drei der Reste $R^2$ bis $R^6$ für den Rest

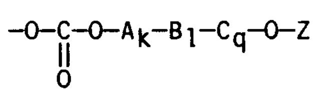

stehen, worin A, B und C für einen Alkylen-, Cycloalkylen-, Oxaalkylen-, Polyoxaalkylen oder Arylenrest, k und l für 1 bis 80 stehen und die Endgruppe Z für Alkyl, Aryl, Alkoxycarbonyl, Alkoxycarbonyloxy oder Aryl steht.

Diese Verbindungen eigenen sich als strahlungsempfindliche Dispersionsemulgatoren.

EP 0 499 836 A2

Die Erfindung betrifft Carbonat- und Carbonylgruppen enthaltende Verbindungen, Verfahren zu ihrer Herstellung sowie deren Verwendung als strahlungsempfindliche Emulgatoren für Dispersionen.

UV-empfindliche Aceto- und Benzophenone werden häufig als Photoinitiatoren in strahlenhärtbaren Dispersionen für Lacke und Überzüge verwendet.

Verbindungen der Formeln

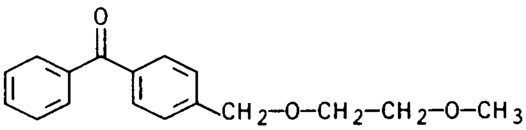

(vgl. US-A 41 99 420),

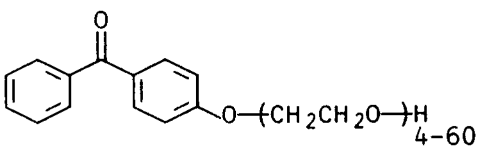

und

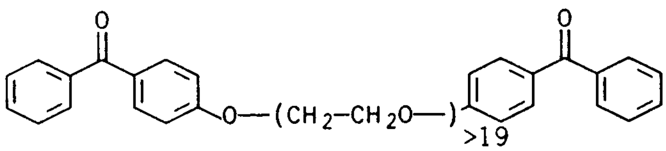

sind bereits aus der US-A 46 02 097 bekannt.

Beim "Uvecryl® P36", einem Handelsprodukt der Fa. UCB, trennt ein besonders langer Spacer aus vier Ethylenoxyeinheiten das Benzophenon vom Acryloxy-Rest.

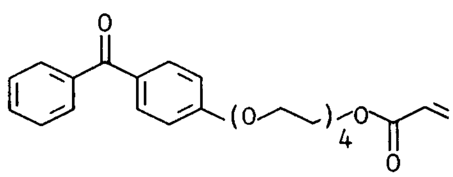

Diese z.B. im Technical Bulletin 2480/885 (1985) der Fa. UCB oder in New Polym. Mat. 1, 63 (1987) beschriebene Verbindung kann in Photopolymeren für Überzugsmassen eingesetzt werden.

Weitere Beispiele für geeignete Verbindungen des genannten Typs sind in EP-A-108 037, JP 5-90 20 313 und GB-PS 2 100 722 beschrieben.

Alle beschriebenen Verbindungen sind entweder mit den dem heutigen Stand der Technik entsprechenden Monomeren nicht dispergierbar oder sie sind photochemisch wenig reaktiv.

Aufgabe der vorliegenden Erfindung ist es, Carbonat- und Carbonylgruppen enthaltende Verbindungen und deren Synthese aufzuzeigen, die sich als strahlungsempfindliche Dispersionsemulgatoren eignen.

Überraschend wurde gefunden, daß die Dispergierbarkeit und die photochemische Aktivität erheblich gesteigert werden können, wenn eine Carbonatgruppe den als Chromophor wirkenden Initiatorteil und den Polyoxyalkylenrest verbindet.

Gegenstand der vorliegenden Erfindung sind Carbonat- und Carbonylgruppen enthaltende Verbindungen der allgemeinen Formel (I)

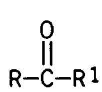

(I),

worin

R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest mit 6 bis 20 Kohlenstoffatomen oder den Rest $R^1$ steht und

$R^1$ für den Rest

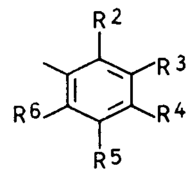

steht, wobei die Reste $R^2$ bis $R^6$ untereinander gleich oder verschieden sind und für H, Alkyl mit 1 bis 4 Kohlenstoffatomen wie z.B. Methyl, Ethyl, Isopropyl, Butyl, Phenyl, OH, $OCH_3$, $OC_2H_5$, SH, $SCH_3$, $SC_2H_5$, $SO_3H$, $SO_3^{\ominus}$, F, Cl, Br, CN, COOH, $COO^{\ominus}$, COOAlkyl mit Alkyl enthaltend 1 bis 17 Kohlenstoffatome, COOAryl, $CF_3$, $N(Alkyl)_2$, N(Alkyl)(Aryl), jeweils mit Alkyl enthaltend 1 bis 4 Kohlenstoffatome oder $N(Aryl)_2$ jeweils mit Aryl enthaltenden 6 bis 20 Kohlenstoffatomen, wie z.B. Phenyl, Tosyl, Xylyl, Naphthyl stehen, mit der Maßgabe, daß $R^2$ und $R^6$ nicht für OH, SH, primäre oder sekundäre Alkylreste stehen und mindestens einer, aber maximal drei der Reste $R^2$ bis $R^6$ für den Rest

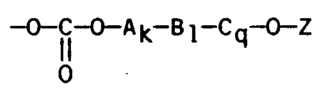

stehen, worin A, B und C untereinander gleich oder verschieden sind und für zweiwertige, gegebenenfalls substituierte, Alkylenrest mit 1 bis 6 Kohlenstoffatomen, für Reste

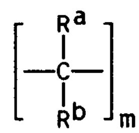

wobei $R^a$ und $R^b$ untereinander gleich oder verschieden sein können und H, OH, Aryl, COOH, $COOCH_3$, $COOC_2H_5$, $SO_3H$ oder Alkyl mit 1 bis 4 Kohlenstoffatomen bedeuten können, für zweiwertige Oxaalkylenreste der Formel

$\{CHR^a\text{-}CHR^b\text{-}O\}_y$

mit y = 1 bis 80 und $R^a$ und $R^b$ mit der oben angegebenen Bedeutung, für Reste

$-(CH_2)_n-O-(CH_2)_p-$ mit n = 1 bis 5 und p = 1 bis 5

oder einen Polyoxaalkylenrest mit 2 bis 20 Sauerstoffatomen, die miteinander über mindestens eine $-CH_2-$, oder $-CH_2-CH(CH_3)-$Gruppe verbunden sind, für einen Rest $-(CH_2)_m-O-CO-O-(CH_2)_n-$, $-(CH_2)_n-O-CO-NH-(CH_2)_m-$, $-(CH_2)_n-NH-CO-O-(CH_2)_m-$, $-(CH_2)m-CO-O-(CH_2)_n-$ oder $-(CH_2)_m-O-CO-(CH_2)_n-$ mit m = 1 bis 10, n = 1 bis 10, für einen gegebenenfalls substituierten Cycloalkylenrest mit 5 bis 10 Kohlenstoffatomen, für einen (Bis)methylencycloalkylenrest mit 6 bis 12 Kohlenstoffatomen oder für einen, gegebenenfalls substituierten, o-, m- oder p-Phenylenrest stehen, k, l und q Laufzahlen von 1 bis 80 bedeuten und $A_k$ und $C_q$ auch für Einfachbindungen stehen können, wobei mindestens einer der Reste $A_k$, $B_l$ und $C_q$ mindestens zwei Sauerstoffatome enhält,

Z für H, Alkyl mit jeweils 1 bis 6 Kohlenstoffatomen in der Alkylgruppe (z.B. i-Propyl oder tert.-Butyl), Phenyl, mit 1 bis 20 Kohlenstoffatome enthaltendem geradkettigen oder verzweigten Alkyl substituiertes Phenyl, oder

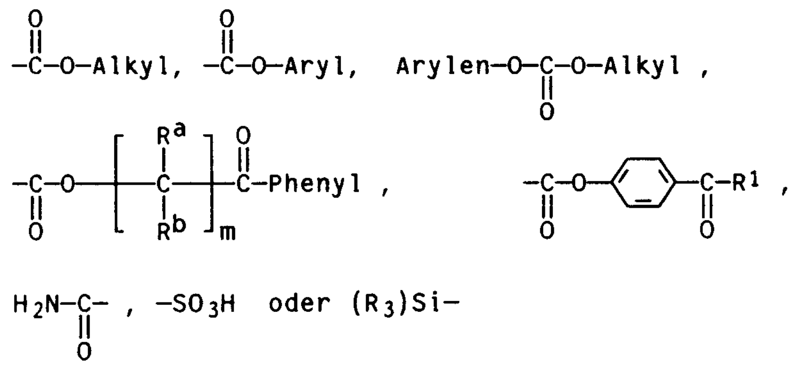

steht oder für den Fall, daß R für einen Arylrest steht, einer der Reste $R^2$ oder $R^6$ für ein Schwefelatom stehen kann, durch das der Arylrest in ortho-Position mit $R^1$ verbunden ist.

Überraschenderweise zeigen die erfindungsgemäßen Verbindungen eine besonders hohe photochemische Reaktivität im kurz- bis längerwelligen UV-Bereich von 254 bis 400 nm, eine gute Lagerstabilität, sowie eine hervorragende Dispergierbarkeit.

Gegenstand der vorliegenden Erfindung ist auch ein verfahren zur Herstellung von Verbindungen der allgemeinen Formel (I), das dadurch gekennzeichnet ist, daß man eine Verbindung der allgemeinen Formel (II)

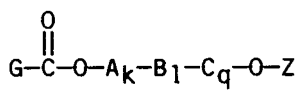

(II),

worin A, B, C, k, l, q und Z die in Anspruch 1 angegebene Bedeutung haben und

G für eine der Gruppen Tosylat, Alkoxy mit 1 bis 5 Kohlenstoffatomen, Halogen, wie z.B. Chlor oder Brom, Imidazolyl, Pyrazolyl, Phosphonium-, Sulfonium-, Ammonium- oder Pyridinium-Kation steht, mit einer Verbindung der allgemeinen Formel (IIIa)

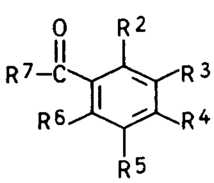

(IIIa),

worin $R^2$ bis $R^6$ die oben angegebene Bedeutung haben, und

$R^7$ für einen geradkettigen Alkylrest mit 1 bis 4 Kohlenstoffatomen, vorzugsweise Methyl, Ethyl, n-Propyl, einen verzweigten, gegebenenfalls substituierten Alkylrest mit 3 oder 4 Kohlenstoffatomen wie i-Propyl, sek.-Hydroxyisopropyl, sek.-Dimethylaminopropyl, sek.-Morpholinopropyl, tert.-Butyl, oder einen Arylrest mit 6 bis 20 Kohlenstoffatomen, wie z.B. Phenyl, Tolyl, Naphthyl, steht, mit der Maßgabe, daß mindestens einer der Reste $R^2$ bis $R^6$ für eine Hydroxylgruppe steht, im äquimolaren Verhältnis oder entsprechend der Anzahl der Hydroxylgruppen in den Resten $R^2$ bis $R^6$ dem Zwei- oder Dreifachen davon, gegebenenfalls in Gegenwart eines inerten Lösungsmittels oder Lösungsmittelsgemisches und eines basischen Katalysators, bei Temperaturen von 0 bis 100 ° C unter wasserfreien Bedingungen miteinander umsetzt.

Gegenstand der vorliegenden Erfindung ist ebenso ein Verfahren zur Herstellung von Verbindungen der allgemeinen Formel (I), das dadurch gekennzeichnet ist, daß man eine Verbindung der allgemeinen Formel (IV)

$HO-A_k-B_l-C_q-O-Z$ (IV),

worin A, B, C, k, l, q und Z die in Anspruch 1 angegebene Bedeutung haben, mit einer Verbindung der

allgemeinen Formel (IIIb)

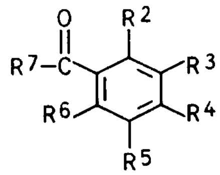 (IIIb),

worin $R^2$ bis $R^6$ die in Anspruch 1 angegebene Bedeutung haben, und

$R^7$ für einen geradkettigen Alkylrest mit 1 bis 4 Kohlenstoffatomen,m einen verzweigten, gegebenenfalls substituierten, Alkylrest mit 3 oder 4 Kohlenstoffaotmen oder einen Arylrest mit 6 bis 20 Kohlenstoffatomen steht, mit der Maßgabe, daß mindestens einer der Reste $R^2$ bis $R^6$ für eine Gruppe

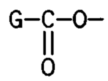 ,

worin G für Tosylat, Alkoxy mit 1 bis 5 Kohlenstoffatomen, Halogen, wie z.B. Chlor oder Brom, z.B. die Chlorformiate von Lutensol® AP20 und Pluronic® PE 6400, Imidazolyl, Pyrazolyl, Phosphonium-, Sulfonium-, Ammonium- oder Pyridinium-Kation steht, im äquimolaren Verhältnis oder entsprechend der Anzahl der Gruppen

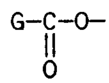

in den Resten $R^2$ bis $R^6$ dem Zwei- oder Dreifachen davon, gegebenenfalls in Gegenwart eines inerten Lösungsmittels oder Lösungsmittelgemisches und eines basischen Katalysators, bei Temperaturen von 0 bis 100 ° C unter wasserfreien Bedingungen miteinander umsetzt.

Für das erstgenannte Verfahren ist bevorzugt, daß als Verbindungen der allgemeinen Formel (II) Chlorformiate von mit Ethylenoxid kettenverlängerten Alkylphenolen oder Bischlorformiate der von Ethylenoxid und/oder Propylenoxid abgeleiteten Oxalkohole eingesetzt werden.

Im Falle des zweitgenannten Verfahrens werden als Verbindungen der allgemeinen Formel (IIIb) gegebenenfalls substituiertes Chlorformylacetophenon, Chlorformylbenzophenon oder Chlorformylthioxanthon eingesetzt.

Für die erfindungsgemäßen Verfahren ist es vorteilhaft, wenn mindestens eine äquimolare Menge einer starken, nicht nucleophilen Base, vorzugsweise eines tertiären Amins, anwesend ist und die Reaktionstemperatur im Bereich von 20 bis 60 ° C liegt.

Die erfindungsgemäßen Verfahren werden vorzugsweise in einem inerten, wasserfreien Lösungsmittel, gegebenenfalls unter Feuchtigkeitsausschluß, durchgeführt.

Die Synthese von Arylcarbonaten ohne copolymerisationsfähige Endgruppe ist bekannt (JP-OS 59-001 438, JP-OS 59-170 033). Eine gute Übersicht geben:

a) Houben-Weyl, Methoden der Organischen Chemie, Bd. 8, S. 75, 101-107, Thieme-Verlag 1952, b) Kirk-Othmer, Encyclopedia of Chemical Technology, Bd. 4, S. 758-771, John Wiley 1978 und c) Ullmann's Encyclopedia of Industrial Chemistry, Bd. A5, S. 197-202, Verlag Chemie 1986.

Das wichtigste Darstellungsverfahren für Carbonate ist die Umsetzung von Kohlensäureesterchloriden mit Alkoholen. Die Prozedur ist ausführlich in Houben-Weyl, Bd. 8 (s.o.) und in der DE-PS 1 080 546 sowie in J.Org.Chem. 26, 5119 (1961) beschrieben. Die Kohlensäureester entstehen in guten bis sehr guten Ausbeuten, wenn der Alkohol und der Chlorkohlensäureester im Molverhältnis 1:1 ohne Solvens oder in überschüssigem Alkohol als Lösungsmittel miteinander zur Reaktion gebracht werden. In den Fällen, in denen der Alkohol bzw. das Phenol und/oder der Chlorkohlensäureester als Feststoff vorliegen, verwendet man aprotische Lösungsmittel, wie z.B. Dichlormethan, Dichlorethan, Acetonitril, Toluol, Xylol usw. Dabei ist hervorzuheben, daß bei den genannten Literaturbeispielen ausschließlich Alkohole mit maximal 10 Kohlenstoffatomen verwendet wurden.

Die als Ausgangsmaterial benötigten Hydroxyacetophenone und Hydroxybenzophenone sind nach

bekannten Verfahren herstellbar. So erhält man beispielsweise 4-Hydroxybenzophenon in ca. 90 %iger Ausbeute durch Friedel-Crafts-Acylierung von Phenol mit Benzoylchlorid in Nitrobenzol in Gegenwart von $AlCl_3$ oder $TiCl_4$ (Houben-Weyl 7/2a, S. 186) oder isomerenfrei durch Oxidation von 4-Hydroxy-diphenylmethan mit 5,6-Dichlor-2,3-dicyan-p-benzochinon (Houben-Weyl 7/2a, S. 681).

Die Synthesen der aminosubstituierten Benzophenone, wie z.B. 2-Benzyl-2-(dimethylamino)-1-(4-hydroxyphenyl)-butan-1-on oder 1-(4-Hydroxyphenyl)-2-methyl-2-morpholino-propan-1-on sind in EP-A-284 561 und EP-A-117 233 beschrieben.

Das 2-Hydroxythioxanthon kann nach dem in den GB-PS 2 108 487 (1981) und GB-PS 2 108 979 (1982) beschriebenen Verfahren aus Thiosalicylsäure und Phenol dargestellt werden.

Die aromatischen Chlorformiate (vgl. J.Prakt.Chem. 313, 331 (1971), dito 317, 62, 73, 81 (1975)) der allgemeinen Formel (IIIb) lassen sich aus einem substituierten Phenol, z.B. 4-Chlor-5'-fluor-2'-hydroxybenzophenon, 4-Chlor-4'-hydroxybenzophenon, 4,4'-Dihydroxybenzophenon, 4-Fluor-4'-hydroxybenzophenon, 4-Hydroxybenzophenon, 3-Hydroxy-thioxanthon, (4-Hydroxyphenyl)-2-hydroxy-2-propylketon (DE-OS 35 34 645) durch Phosgenierung nach literaturbekannten Standardverfahren mit Phosgen, s. z.B. Houben-Weyl, Methoden der Organischen Chemie, Bd. 8, Thieme-Verlag 1952, Trichlormethylchlorformat (Diphosgen), J.Prakt.Chem. 126, 210 (1930), dito 128, 233 (1930), Chem.Abstr. 95, 81766, J.Org.Chem. 50, 715 (1985), J.Org.Chem. 41, 2070 (1976), Angew. Chem. 89, 267 (1977), dem kristallinen Triphosgen, Angew. Chem. 99, 922 (1987), N,N'-Carbonyldiimidazol oder N,N'-Carbonyldi-s-triazol (Fieser 1, 116 (1967)), in guten Ausbeuten herstellen.

Über den Einsatz alternativer Verfahren für die Phosgenierung, z.B. die Umsetzung mit Chlorkohlensäureestern gibt "Merck Kontakte" 1981 (1), 14-18 Auskunft.

Für die Synthese der erfindungsgemäßen Verbindungen werden die entsprechend substituierten Mono- bzw. Dialkohole benötigt. Beispielhaft für diese Verbindungen seien genannt: Lutensol® AP 20 (= Isononylphenolethylenoxidaddukt mit im Mittel 20 Ethylenoxideinheiten),

Pluronic® PE 6400 (= Ethylenoxid/Propylenoxid-Blockcopolymer, $\overline{M}_w$ ca. 2900).

Beispiele für als Ausgangsprodukte geeignete Chlorformiate sind z.B.

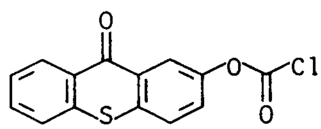

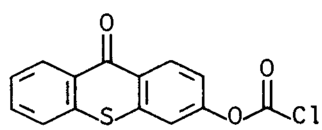

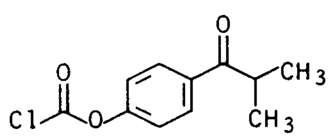

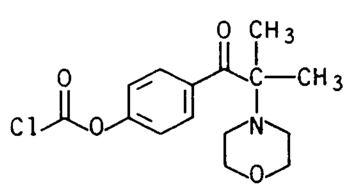

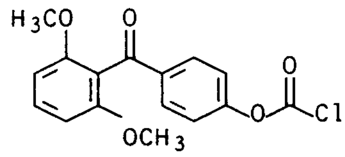

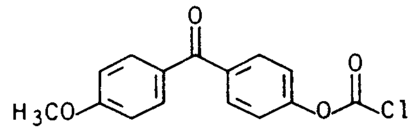

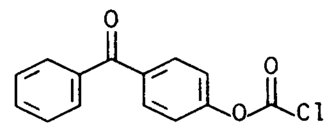

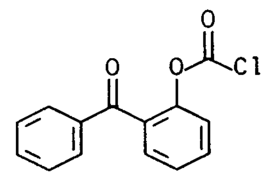

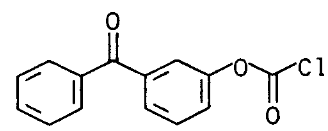

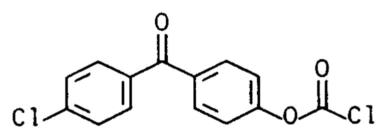

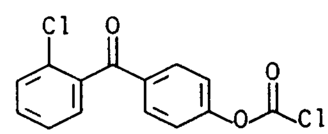

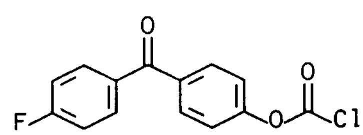

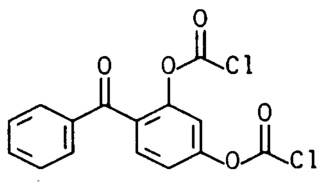

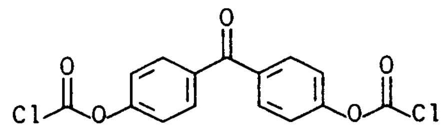

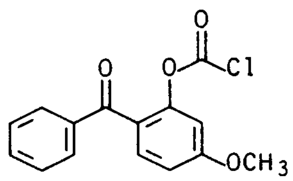

Die Umsetzung erfolgt im äquimolaren Verhältnis (gegebenenfalls mit bis zu 10 bis 30 %igem Überschuß) oder entsprechend der Anzahl der Gruppen G-CO-O- in den Resten $R^8$ bis $R^{12}$ dem Zwei- oder Dreifachen davon unter Feuchtigkeitsausschluß, gegebenenfalls in Gegenwart eines inerten Lösungsmittels oder Lösungsmittelgemisches und eines basischen Katalysators bei Temperaturen von 0 bis 100 °C, vorzugsweise bei 20 bis 50 °C, miteinander umsetzt.

Für die Umsetzung der Hydroxyaceto-, -benzophenone bzw. -thioxanthone werden im allgemeinen die entsprechenden Chlorformiate benötigt. Diese sind nach literaturbekannten Verfahren, wie z.B. in Eur. Polym. J. 14, 205 (1978); J.Polym.Sci.Polym.Symp. 66, 41 (1979); Bull.Soc.Chim. Belg. 93, 159 (1984) beschrieben, bequem und in guten Ausbeuten darstellbar.

Überraschend wurde gefunden, daß die erfindungsgemäßen Aceto- und Benzophenon- sowie Thioxanthonderivate leicht und in sehr guter Ausbeute zugänglich sind.

Zum Herstellverfahren ist im einzelnen Folgendes auszuführen.

Die bei der Reaktion eingesetzten Chlorformiate reagieren leicht mit Nucleophilen, u.a. auch mit Wasser. Deshalb ist bei der Reaktion auf Feuchtigkeitsausschluß durch Verwendung getrockneter nicht nucleophiler Lösungsmittel, wie z.B. Acetonitril, Dichlormethan, Dichlorethan, THF, Toluol, Xylol, Chlorbenzol, Essigester, Chloroform usw. und gegebenenfalls auf den Aufbau einer Inertgasatmosphäre, z.B. Stickstoff, Argon oder Kohlendioxid zu achten.

In der Regel wird eine Lösung oder Suspension der Hydroxyverbindung in einem inerten Lösungsmittel, welches auch wegfallen kann, wenn die Verbindung bei der Reaktionstemperatur flüssig ist, bei Temperaturen von 0 bis 100 °C, vorzugsweise bei 10 bis 50 °C, in Gegenwart eines basischen, nicht nucleophilen Amins, vorzugsweise Triethylamin, 4-Dimethylaminopyridin, Imidazol, 1,4-Diazabicyclo[2.2.2]octan, 1,5-Diazabicyclo[4.3.0]non-5-en, 1,8-Diazabicyclo[5.4.0]undec-7-en, Polyvinylpyridin, N,N'-Dimethylpropylenharnstoff, N,N'-Dimethylethylenharnstoff usw., vorgelegt. Dann wird die Chlorformylverbindung, u.U. gelöst in einem inerten Lösungsmittel, wie z.B. Dichlormethan, Dichlorethan, Acetonitril, Toluol, Chlorbenzol, Xylol usw. unter Rühren im oben angegebenen Temperaturbereich zugetropft. Diese Arbeitsweise eignet sich besonders für größere Ansätze.

In analoger Weise können anstelle der Chlorformiate auch die Tosylate, Imidazolyl, Pyrazolyl, Phosphonium-, Sulfonium-, Ammonium- oder Pyridinium-Verbindungen für die Synthese eingesetzt werden.

Nach einer Nachrührzeit von 1 bis 48 Stunden, vorzugsweise 1 bis 20 Stunden bei 10 bis 40 °C wird nach Standardverfahren filtriert, gewaschen, getrocknet und das Produkt nach dem Umkristallisieren, Destillieren oder Extrahieren isoliert.

Die erfindungsgemäßen Acetophenone, Benzophenone und Thioxanthone eignen sich als strahlenhärtbare Emulgatoren für wäßrige Polymerdisperionen. Sie lagern sich bevorzugt an der Oberfläche der Polymerteilchen an und stehen damit für Photovernetzungsreaktionen zur Verfügung (vgl. Patentanmeldung P .. .. ..., O.Z. 0050/42229).

Für den Fall, daß die erfindungsgemäßen Carbonat- und Carbonylgruppen enthaltenden Verbindungen

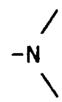

Strukturen enthalten, ist es zweckmäßig, sie mit aromatischen Ketonen, wie Benzophenon oder Acetophenon zu kombinieren.

Außerdem kann es von Vorteil sein, die erfindungsgemäßen Carbonat- und Carbonylgruppen enthaltenden Verbindungen mit Aminen, bevorzugt tertiären Aminen, wie z.B. Triethylamin, Triethanolamin oder Diethylethanolamin einzusetzen, um die Strahlungsempfindlichkeit zu erhöhen.

Die in den Beispielen genannten Teile und Prozente sind, soweit nicht anders angegeben, Gewichtsteile und Gewichtsprozente. Für alle in den folgenden Beispielen angegebenen Verbindungen wurde die Struktur z.T. durch unabhängige Synthesen und in allen Fällen durch korrekte $^1$H-NMR-, IR- und Massenspektren sowie durch übereinstimmende Elementaranalysen bestätigt.

Beispiele für die Herstellung von als Ausgangsprodukte geeigneten Chlorformylverbindungen:

1. 4-Chlorformylbenzophenon

In eine Lösung von 4 kg 4-Hydroxybenzophenon und 190 g Benzyltrimethylammoniumchlorid in 11,4 kg o-Xylol wurden in 5 Stunden insgesamt 3,4 kg Phosgen eingeleitet. Die Innentemperatur wurde während dieser Zeit von 95 auf 120 °C erhöht. Nach Beendigung der Phosgeneinleitung wurde 30 Minuten bei 115 °C nachgerührt. Zur Aufarbeitung wurde der Phosgenüberschuß mit Stickstoff ausgetrieben. Das gegen Ende der Reaktion ausgefallene Salz (Katalysator) wurde abfiltriert und das Lösungsmittel abdestilliert. Man erhielt 4,9 kg (93 %) gelblich gefärbtes 4-Chlorformylbenzophenon vom Schmp. 67-72 °C. Dieses Rohprodukt mit Cl-Wert = 12,69 % (theor. 13,60 %) wurde direkt ohne weitere Reinigung für die Folgereaktionen verwendet.

In Analogie zu der im Beispiel 1 genannten Vorschrift wurden die folgenden Chlorformylverbindungen hergestellt:

2. 2-Chlorformyl-thioxanthon

Aus 2-Hydroxythioxanthon wurden in 79 % Ausbeute 2-Chlorformylthioxanthon (Cl: Ber. = 12,19 %; Gef. = 12,03 %) erhalten.

3. 3-Chlorformylthioxanthon

3-Chlorformylthioxanthon war in 63 % Ausbeute aus 3-Hydroxythioxanthon (Cl: Ber. = 12,19 %; Gef. = 11,22 %) zugänglich.

4. (4-Chlorformylphenyl)-(2-hydroxy-2-propyl)keton

Aus (4-Hydroxyphenyl)-(2-hydroxy-2-propyl)keton wurden in 75 %iger Ausbeute (4-Chlorformylphenyl)-(2-hydroxy-2-propyl)keton als Rohprodukt (Cl: Ber. = 14,61 %; Gef. = 13,07 %) erhalten.

5. Aus 1-(4-Hydroxyphenyl)-2-methyl-2-morpholinopropan-1-on wurde durch Phosgenierung zunächst das Hydrochlorid erhalten, welches nach vorsichtigem Versetzen mit 1,5-Diazabicyclo[4.3.0]non-5-en in das freie Amin überführt werden konnte; Ausbeute: 62 % (Cl: Ber. = 11.37 %; Gef. = 11.21 %).

Herstellung erfindungsgemäßer Dispersionsemulgatoren:

Beispiel 1

Reaktion von 4-Chlorformylbenzophenon mit Lutensol® AP 20

275 g (0,25 mol) Lutensol® AP 20 und 30 g (0,3 mol) Triethylamin wurden in 1000 ml Toluol bei 25 °C gelöst. In 30 min wurden dann 75 g (0,25 mol) 4-Chlorformylbenzophenon gelöst in 150 ml Toluol zugetropft und 15 Std. bei RT nachgerührt. Das ausgefallene Triethylaminhydrochlorid wurde sorgfältig abgesaugt und das Filtrat bis zur Gewichtskonstanz im Ölpumpenvakuum eingeengt.

Ausbeute: 318 g (96 %) farblose, viskose Flüssigkeit

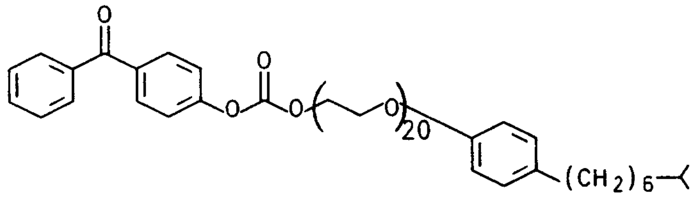

Zu dem gleichen Produkt gelangt man in einer Ausbeute von 85 %, wenn 4-Hydroxybenzophenon und phosgeniertes Lutensol AP 20 unter den genannten Bedingungen umgesetzt und aufgearbeitet werden.

In Analogie zu Beispiel 1 wurden die folgenden unsymmetrischen Carbonate hergestellt.

Beispiele 2 - 10

| Beispiel | Chlorformiat | Lutensol® | Ausbeute |
|---|---|---|---|
| 2 | 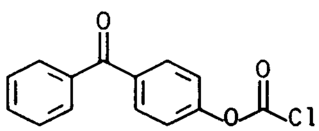 | AP 6 | 87 % |
| 3 | | AP 10 | 92 % |
| 4 | 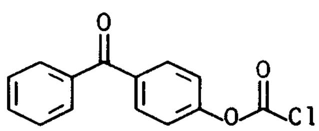 | AP 6 | 95 % |
| 5 | | AP 10 | 94 % |
| 6 | | AP 20 | 97 % |
| 7 | 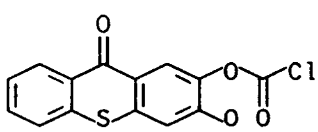 | AP 20 | 82 % |
| 8 | 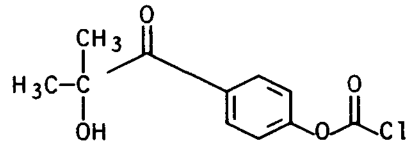 | AP 6 | 97 % |
| 9 | | AP 10 | 90 % |
| 10 | | AP 20 | 87 % |

Beispiel 11

Umsetzung von 4-Chlorformylbenzophenon mit Pluronic® PE 6400

In 1000 ml Toluol wurden bei RT 363 g (0.125 mol) Pluronic PE 6400 und 30 g (0,3 mol) Triethylamin vorgelegt und anschließend bei 25 °C eine Lösung von 75 g (0,25 mol) 4-Chlorformylbenzophenon in 150 ml Toluol zugetropft. Das Reaktionsgemisch wurde 17 Std. nachgerührt, auf ca. 10 °C abgekühlt, sorgfältig filtriert und im Ölpumpenvakuum bis zur Gewichtskonstanz eingeengt.

Ausbeute: 412 g (98 %) klare, farblose, viskose Flüssigkeit.

Die $^1H$-, $^{13}C$-NMR und IR-Spektren bestätigen die Struktur

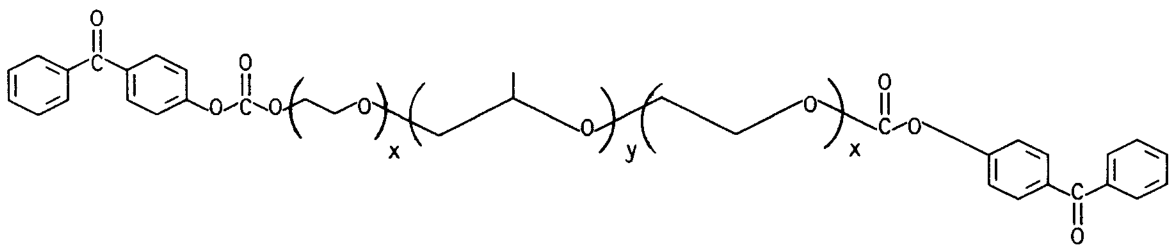

**Patentansprüche**

**1.** Carbonat- und Carbonylgruppen enthaltende Verbindungen der allgemeinen Formel (I)

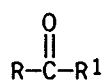 (I),

worin

R für einen geradkettigen Alkylrest mit 1 bis 4 Kohlenstoffatomen, einen verzweigten, gegebenenfalls substituierten, Alkylrest mit 3 oder 4 Kohlenstoffatomen, für einen Arylrest mit 6 bis 20 Kohlenstoffatomen oder den Rest $R^1$ steht und

$R^1$ für den Rest

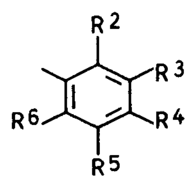

steht, wobei die Reste

$R^2$ bis $R^6$ untereinander gleich oder verschieden sind und für H, Alkyl mit 1 bis 4 Kohlenstoffatomen, Phenyl, OH, $OCH_3$, $OC_2H_5$, SH, $SCH_3$, $SC_2H_5$, $SO_3H$, $SO_3^{\ominus}$, F, Cl, Br, CN, COOH, $COO^{\ominus}$, COOAlkyl mit Alkyl enthaltend 1 bis 17 Kohlenstoffatome, COOAryl, $CF_3$, $N(Alkyl)_2$, N(Alkyl)(Aryl), jeweils mit Alkyl enthaltend 1 bis 4 Kohlenstoffatome oder $N(Aryl)_2$ jeweils mit Aryl enthaltend 6 bis 20 Kohlenstoffatomen stehen mit der Maßgabe, daß $R^2$ und $R^6$ nicht für OH, SH, primäre oder sekundäre Alkylreste stehen und mindestens einer, aber maximal drei der Reste $R^2$ bis $R^6$ für den Rest

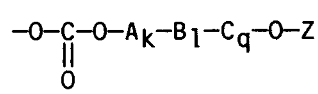

stehen, worin A, B und C untereinander gleich oder verschieden sind und für zweiwertige, gegebenenfalls substituierte, Alkylenrest mit 1 bis 6 Kohlenstoffatomen, für Reste

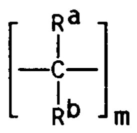

mit m = 1 bis 6,
wobei $R^a$ und $R^b$ untereinander gleich oder verschieden sein können und H, OH, Aryl, COOH, $COOCH_3$, $COOC_2H_5$, $SO_3H$ oder Alkyl mit 1 bis 4 Kohlenstoffatomen bedeuten können,
für zweiwertige Oxaalkylenreste der Formel

$-(CHR^a-CHR^b-O)_y-$

mit y = 1 bis 80 und $R^a$ und $R^b$ mit der oben angegebenen Bedeutung, für Reste

$-(CH_2)_n-O-(CH_2)_p-$ mit n = 1 bis 5 und p = 1 bis 5

oder einen Polyoxaalkylenrest mit 2 bis 20 Sauerstoffatomen, die miteinander über mindestens eine $-CH_2-$, oder $-CH_2-CH(CH_3)$-Gruppe verbunden sind, für einen Rest $-(CH_2)_m-O-CO-O-(CH_2)_n-$, $-(CH_2)_n-O-CO-NH-(CH_2)_m-$, $-(CH_2)_n-NH-CO-O-(CH_2)_m-$, $-(CH_2)_m-CO-O-(CH_2)_n-$ oder $-(CH_2)_m-O-CO-(CH_2)_n-$ mit m = 1 bis 10, n = 1 bis 10, für einen, gegebenenfalls substituierten, Cycloalkylenrest mit 5 bis 10 Kohlenstoffatomen, für einen (Bis)methylencycloalkylenrest mit 6 bis 12 Kohlenstoffatomen oder für einen, gegebenenfalls substituierten, o-, m- oder p-Phenylenrest stehen, k, l und q Laufzahlen von 1 bis 80 bedeuten und $A_k$ und $C_q$ auch für Einfachbindungen stehen können, wobei mindestens einer der Reste $A_k$, $B_l$ und $C_q$ mindestens zwei Sauerstoffatome enthält,

Z für H, Alkyl mit jeweils 1 bis 6 Kohlenstoffatomen in der Alkylgruppe, Phenyl, mit 1 bis 20 Kohlenstoffatome enthaltendem geradkettigen oder verzweigten Alkyl substituiertes Phenyl, oder

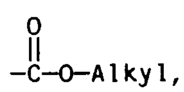 , 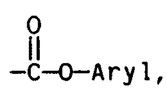 , 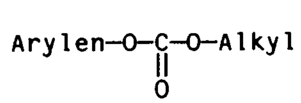 ,

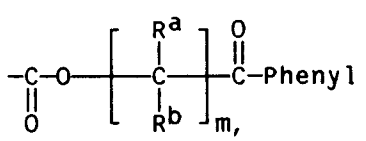 , 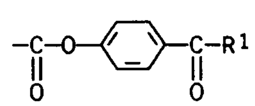 ,

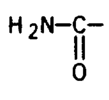 , $-SO_3H$ oder $(R_3)Si-$

steht oder für den Fall, daß R für einen Arylrest steht, einer der Reste $R^2$ oder $R^6$ für ein Schwefelatom stehen kann, durch das der Arylrest in ortho-Position mit $R^1$ verbunden ist.

**2.** Verfahren zur Herstellung von Verbindungen der allgemeinen Formel (I), dadurch gekennzeichnet, daß man eine Verbindung der allgemeinen Formel (II)

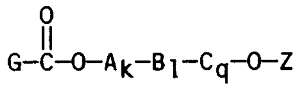

(II),

worin A, B, C, k, l, q und Z die in Anspruch 1 angegebene Bedeutung haben und

G für eine der Gruppen Tosylat, Alkoxy mit 1 bis 5 Kohlenstoffatomen, Halogen, Imidazolyl, Pyrazolyl, Phosphonium-, Sulfonium-, Ammonium- oder Pyridinium-Kation steht,

mit einer Verbindung der allgemeinen Formel (IIIa)

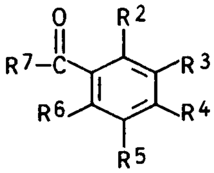

(IIIa),

worin $R^2$ bis $R^6$ die in Anspruch 1 angegebene Bedeutung haben, und

$R^7$ für einen geradkettigen Alkylrest mit 1 bis 4 Kohlenstoffatomen, einen verzweigten, gegebenenfalls substituierten Alkylrest mit 3 oder 4 Kohlenstoffatomen oder einen Arylrest mit 6 bis 20 Kohlenstoffatomen steht, mit der Maßgabe, daß mindestens einer der Reste $R^2$ bis $R^6$ für eine Hydroxylgruppe steht, im äquimolaren Verhältnis oder entsprechend der Anzahl der Hydroxylgruppen in den Resten $R^2$ bis $R^6$ dem Zwei- oder Dreifachen davon, gegebenenfalls in Gegenwart eines inerten Lösungsmittels oder Lösungsmittelgemisches und eines basischen Katalysators, bei Temperaturen von 0 bis 100 °C unter wasserfreien Bedingungen miteinander umsetzt.

**3.** Verfahren zur Herstellung von Verbindungen der allgemeinen Formel (I), dadurch gekennzeichnet, daß man eine Verbindung der allgemeinen Formel (IV)

$HO-A_k-B_l-C_q-O-Z$ (IV),

worin A, B, C, k, l, q und Z die in Anspruch 1 angegebene Bedeutung haben, mit einer Verbindung der allgemeinen Formel (IIIb)

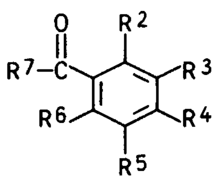

(IIIb),

worin $R^2$ bis $R^6$ die in Anspruch 1 angegebene Bedeutung haben, und

$R^7$ für einen geradkettigen Alkylrest mit 1 bis 4 Kohlenstoffatomen, einen verzweigten, gegebenenfalls substituierten, Alkylrest mit 3 oder 4 Kohlenstoffaotmen oder einen Arylrest mit 6 bis 20 Kohlenstoffatomen steht, mit der Maßgabe, daß mindestens einer der Reste $R^2$ bis $R^6$ für eine Gruppe

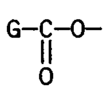

,

worin G für Tosylat, Alkoxy mit 1 bis 5 Kohlenstoffatomen, Halogen, Imidazolyl, Pyrazolyl, Phosphonium-, Sulfonium-, Ammonium- oder Pyridinium-Kation steht, im äquimolaren Verhältnis oder entsprechend der Anzahl der Gruppen

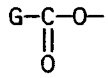 ,

in den Resten $R^2$ bis $R^6$ dem Zwei- oder Dreifachen davon, gegebenenfalls in Gegenwart eines inerten Lösungsmittels oder Lösungsmittelgemisches und eines basischen Katalysators, bei Temperaturen von 0 bis 100 ° C unter wasserfreien Bedingungen miteinander umsetzt.

**4.** Verfahren nach Anspruch 2, dadurch gekennzeichnet, daß als Verbindungen der allgemeinen Formel (II) Chlorformiate von mit Ethylenoxid kettenverlängerten Alkylphenolen oder Bischlorformiate der von Ethylenoxid und/oder Propylenoxid abgeleiteten Oxalkohole eingesetzt werden.

**5.** Verfahren nach Anspruch 3, dadurch gekennzeichnet, daß es sich bei den Verbindungen der allgemeinen Formel (IIIb) um ein gegebenenfalls substituiertes Chlorformylacetophenon, Chlorformylbenzophenon oder Chlorformylthioxanthon handelt.

**6.** Verfahren nach einem der vorhergehenden Ansprüche, dadurch gekennzeichnet, daß mindestens eine äquimolare Menge einer starken, nicht nucleophilen Base, vorzugsweise eines tertiären Amins, anwesend ist.

**7.** Verfahren nach einem der vorhergehenden Ansprüche, dadurch gekennzeichnet, daß die Reaktionstemperatur im Bereich von 20 bis 60 ° C liegt.

**8.** Verfahren nach einem der vorhergehenden Ansprüche, dadurch gekennzeichnet, daß in einem inerten, wasserfreien Lösungsmittel, gegebenenfalls unter Feuchtigkeitsausschluß, gearbeitet wird.

**9.** Verwendung der Verbindungen nach Anspruch 1, gegebenenfalls in Gegenwart eines Amins, als strahlungsempfindliche Dispersionsemulgatoren.